# FASCICULE DE BREVET EUROPEEN

(11) **EP 4 045 632 B1**
(45) Date de publication et mention de la délivrance du brevet: **06.12.2023**
(21) Numéro de dépôt: 20804609.4
(22) Date de dépôt: 19.10.2020
(51) Int. Cl.: C12N 5/077, C12M 1/32

(54) **PROCEDE D'OBTENTION DE SPHEROIDES DE CELLULES**
VERFAHREN ZUR HERSTELLUNG VON ZELLSPHÄROIDEN
METHOD FOR OBTAINING CELL SPHEROIDS

(30) Priorité: 17.10.2019 FR 1911597
(43) Date de publication de la demande: 24.08.2022
(73) Titulaire: D.I.V.A Expertise, 31100 Toulouse (FR)
(72) Inventeur: KEOPHIPHATH, Mayoura, 31100 TOULOUSE (FR); BELLES, Chloé, 31100 TOULOUSE (FR); GOMES, Aurélie, 31100 TOULOUSE (FR); NORLUND, Marine, 31100 TOULOUSE (FR)
(74) Mandataire: Regimbeau
(86) Numéro de dépôt international: PCT/FR2020/051878
(87) Numéro de publication internationale: WO 2021/074556

(56) Documents cités:
- SUNG-IL LEE ET AL: "Formation of size-controllable spheroids using gingiva-derived stem cells and concave microwells: Morphology and viability tests", BIOMEDICAL REPORTS MAY 2014 SPANDIDOS PUBLICATIONS GBR, vol. 4, no. 1, 5 novembre 2015 (2015-11-05), pages 97-101, XP055709880, Greece ISSN: 2049-9434, DOI: 10.3892/br.2015.539
- BRANDON D MARKWAY ET AL: "ENHANCED CHONDROGENIC DIFFERENTIATION OF HUMAN BONE MARROW-DERIVED MESENCHYMAL STEM CELLS IN LOW OXYGEN ENVIRONMENT MICROPELLET CULTURES", CELL TRANSPLANTATION,, vol. 19, 1 janvier 2010 (2010-01-01), pages 29-42, XP002785390,
- TONI-MARIE ACHILLI ET AL: "Advances in the formation, use and understanding of multi-cellular spheroids", EXPERT OPINION ON BIOLOGICAL THERAPY, vol. 12, no. 10, 12 juillet 2012 (2012-07-12), pages 1347-1360, XP055369856, ASHLEY, LONDON; GB ISSN: 1471-2598, DOI: 10.1517/14712598.2012.707181
- PAUL A. TURNER ET AL: "Three-Dimensional Spheroid Cell Model of In Vitro Adipocyte Inflammation", TISSUE ENGINEERING PART A, vol. 21, no. 11-12, 1 juin 2015 (2015-06-01), pages 1837-1847, XP055709855, US ISSN: 1937-3341, DOI: 10.1089/ten.tea.2014.0531

## Description

La présente invention concerne le domaine de la culture cellulaire, en particulier, la culture de cellules tridimensionnelle, leurs procédés et utilisations.

Des études toxicologiques sur la base de cultures cellulaires bidimensionnelles ont été utilisées pour examiner les effets des médicaments sur le métabolisme des cellules, l'activité des enzymes, etc.

Bien que la capacité de développer des cellules en couches plates soit simple et permette d'étudier divers aspects de la physiologie cellulaire, elles ne permettent pas de rendre compte de la complexité et de l'architecture d'un organe entier. En particulier, dans un modèle 2D, la matrice extracellulaire, les interactions inter-cellulaires et avec la matrice, permettant de refléter la complexité des fonctions cellulaires, sont absentes.

Les systèmes de culture tridimensionnels peuvent former un tissu fonctionnel présentant des caractéristiques proches des structures in vivo. La culture cellulaire tridimensionnelle permet aux cellules d'interagir avec leur environnement dans les trois dimensions et est et reflète mieux le fonctionnement intégré d'un organe.

Les industries de la santé, de la nutrition, de la cosmétique et de l'esthétique font face à des évolutions de leur environnement. D'une part, l'endurcissement de la réglementation, interdisant par exemple les tests sur animaux en Cosmétique et d'autre part, l'évolution des attentes des consommateurs vers des produits plus efficaces, aux résultats scientifiquement prouvés, motivent les industriels à trouver de nouveaux leviers d'innovation et des outils plus pertinents pour évaluer leurs produits, comprendre leurs mécanismes d'action et optimiser leur efficacité.

D'autres modèles tridimensionnels tels que des modèles sphéroïdes du foie ont été décrits. Les sphéroïdes hépatiques peuvent être composés de plusieurs types de cellules initialement utilisés dans des cultures bidimensionnelles pour tester des molécules xénobiotiques ainsi que leur métabolisation hépatique.

La culture cellulaire 3D peut produire des structures tissulaires et cellulaires spécifiques et imiter les fonctions et les réponses de tissus réels aux stimuli externes et ce d'une manière plus pertinente que les modèles de culture bidimensionnelle.

La production reproductible de sphéroïdes cellulaires 3D est très demandée. Jusqu'à présent, différentes techniques (à base de supports protéiques ou polysaccharidiques (scaffold) ou via des inducteurs chimiques) ont été utilisées pour la formation de sphéroïdes. La technique de recouvrement liquide (LOT) est l'une des méthodologies les plus explorées, en raison de son faible coût et la manipulation facile. Cette technique a été largement étudiée afin d'améliorer son potentiel d'application dans l'analyse à haut débit.

Dans un domaine particulier, et face notamment à la progression des problèmes de surcharge pondérale et au vieillissement accéléré de la population, le tissu adipeux dit graisseux, qui est le siège d'altérations majeures au cours de ces processus physiopathologiques est devenu une des cibles majeures de la recherche industrielle. Les industriels considèrent désormais la recherche sur le tissu adipeux comme un axe d'innovation et souhaitent de plus en plus l'intégrer dans le développement de leurs produits afin d'en tirer un avantage concurrentiel sur le marché.

Le tissu adipeux est un tissu de très grande hétérogénéité cellulaire, renfermant notamment des adipocytes matures responsables du stockage des lipides et de leur libération lors de besoins énergétiques ainsi que des précurseurs de ces cellules, les préadipocytes, qui seront recrutés pour la formation de nouveaux adipocytes et qui participent aux altérations du tissu au cours de l'obésité et du vieillissement.

Les préadipocytes, contrairement aux adipocytes, sont des cellules aux propriétés adhérentes et faciles à cultiver en conditions classiques de culture cellulaire en système bidimensionnel. En revanche, ces conditions de culture en 2D requièrent un nombre important de cellules, des ressources matérielles non négligeables, un temps long de culture (deux semaines) sans permettre une maturation optimale de ces cellules et avec en plus un risque important de décollement de cellules en fin de culture.

Ainsi, le préadipocyte est devenu une cible d'action des produits de santé et de beauté compte tenu de son rôle dans l'expansion de la masse adipeuse et dans les processus fibro-inflammatoires qui caractérisent les dépôts adipeux des personnes en surcharge pondérale, des zones de cellulite ou encore des peaux âgées.

Dans le cas des préadipocytes, les cultures en système 3D existantes intègrent une matrice protéique de nature collagénique le plus souvent ou impliquent des techniques complexes et coûteuses.

Ho & al., PLOS one, 2012, vol. 7, décrivent la formation de sphéroïdes de cellules cancéreuses de cancer du sein par la technique de « liquid overlay cultivation ».

Emon MP et al., Endocrinology ; 2015 Dec, décrit une technique de culture cellulaire en 3D simple et reproductible qui permet de différencier des pré-adipocytes viscéraux de souris en adipocytes. Les pré-adipocytes sont intégrés dans un gel de collagène à une concentration de 300 000 cellules par puits pour 500µl de gel.

Daya S et al., Différentiation, 2007, p360-370, décrit l'utilisation d'un système de culture en trois dimensions pour la culture et la différenciation de pré-adipoyctes de souris contenus dans un gel de collagène. Les cellules ont été ajoutées au gel de manière à contenir 105 cellules / ml de gel puis le mélange est déposé dans une plaque 24- puits. Au fond des puits était déposée une lamelle de verre de 13mm pour retirer plus facilement le gel de collagène à la fin de différenciation.

Louis F, Biotechnol Bioeng, 2017, volume 114, divulgue l'utilisation d'un hydrogel bio-fonctionnalisé contenant des composants de la matrice extracellulaire adipeuse tels que le collagène l, collagène VI et le domaine de liaison cellulaire de la fibronectine, qui permet la culture 3D d'adipocytes matures issus des lignées cellulaires de pré adipocytes, acquérant une organisation de type in vivo, en formant des sphéroïdes multicellulaires d'adipocytes. J.Borges, Mimimally Invasive Therapy & Allied technologies, 2007, volume 16, décrit une méthode de co-culture cellulaire en 3D de préadipocytes et de cellules endothéliales pour permettre l'étude de leurs interactions dans un contexte de prolifération et d'angiogenèse. Les préadipocytes et les cellules endothéliales ont été cultivés en 3D au sein d'une matrice de fibrine dans des plaques 96 puits non adhérents (à fond rond concave) pour permettre leur regroupement en sphéroïdes.

Dans Korff & Augustin, Journal of Cell Biology, 1998, volume 143, la technique utilisée pour générer des sphéroïdes endothéliaux est une méthode chimique. En effet, il est décrit dans le Matériel et Méthodes de ce document que les cellules sont mises en suspension dans des boites de culture, dans du milieu de culture qui contient du méthocel (20%), dans des conditions de températures à 37°C, 5% de CO2 et 100% humidité. Ces conditions permettent l'agrégation des cellules en moins de 4 heures. Il est décrit que « La variation de la concentration en méthocel au cours de la formation de sphéroïdes a donc été utilisée pour contrôler la taille moyenne des sphéroïdes », ce qui confirme le fait que le méthocel est l'agent de formation des sphéroïdes obtenus dans ce document.

Turner, Biotechnologies & Bioengineering, 2013, volume 111, décrit un mode de culture cellulaire en 3D de cellules préadipocytaires qui seront induites en différenciation pour devenir des adipocytes. Cette culture de pré-adipocytes en 3D se fait dans des plaques de 24 puits ronds à fond plat avec un substrat composé d'un polymère artificiellement fabriqué : « elastin-like-polypeptide ELP » associé à un autre polymère synthétique : « polyethyleneimine PEI ».

Bhang & Cho, Biomaterials, 2011, vol. 32 p.2734-374, traite de l'efficacité accrue de l'angiogenèse suite à la greffe de sphéroïdes formés à partir de cellules souches dans un tissus ischémique. Les sphéroïdes sont obtenus en plaçant la culture cellulaire dans une flasque qui subit une agitation d'environ 70 rpm durant 3 jours. Ce document décrit l'obtention de sphéroïdes par un inducteur mécanique qui se trouve être l'agitation de la flasque pendant plusieurs jours.

WO2016069892 A1, décrit un système permettant la culture 3D de cellules telle que des sphéroïdes. Ce système comprend une plaque composée de micro puits ou de puits qui ont une forme permettant la formation de sphéroïdes lorsque des cellules sont en culture, contenues dans un dispositif de culture tel qu'une flasque de culture. De plus ils sont structurés et agencés pour permettre le mouvement du liquide dans et hors du puits sans emprisonner de l'air entre le substrat et le liquide ou gouttelettes lipidiques qui sont introduites dans les puits, grâce à leurs structures capillaires.

De manière générale, les techniques décrites dans l'état de la technique impliquent soit l'utilisation d'inducteurs de sphéroïdisation chimiques, soit de support matriciel (scaffold) exogène, soit de techniques complexes ou longues nécessitant plusieurs jours de manipulation afin d'obtenir les sphéroïdes.

La présente invention vise ainsi la fourniture d'un procédé simple et rapide d'obtention de sphéroïdes de cellules indifférenciées et/ou différenciées, en particulier de sphéroïdes de préadipocytes voire encore de sphéroïdes d'adipocytes.

La présente invention fournit un outil précieux pour étudier l'impact de composés dans un système de culture cellulaire tridimensionnel qui est plus proche, et devrait donc être plus prédictif, de la situation clinique et offre une solution tridimensionnelle unique, simple et peu coûteuse.

C'est ainsi que, selon un premier mode de réalisation, la présente invention fournit un procédé d'obtention de sphéroïdes de cellules indifférenciées comprenant les étapes suivantes :
- a. Ensemencement de cellules indifférenciées isolées et amplifiées dans un contenant dont le fond a un profil arrondi concave, contenant un milieu de culture adapté,
- b. Centrifugation dudit contenant,
- c. Rotation du contenant selon un axe passant perpendiculairement au plan défini par la surface du milieu de culture, la rotation étant de de 160 à 200°, avantageusement 180°,
- d. répétition des étapes b) et c) entre 1 et 15 fois,
- e. Ensemencement et incubation des sphéroïdes de cellules indifférenciées néo formées dans favorisant la formation complète de sphéroïdes, en particulier un milieu supplémenté en sérum foetal.
Selon un mode de réalisation, le procédé est caractérisé en ce que lesdites cellules indifférenciées ne sont pas des cellules embryonnaires humaines.

La première étape consiste en ou comprend l'ensemencement de cellules indifférenciées isolées et amplifiées.

Par l'expression cellules indifférenciées, on entend des cellules indifférenciées ou peu différenciées que l'on trouve au sein de tissus qui sont composés en majorité de cellules différenciées dans la plupart des tissus et organes adultes. Les cellules indifférenciées s'opposent ainsi aux cellules totalement différenciées. Ainsi, par exemple, dans le contexte de l'invention, des adipocytes sont des cellules totalement différenciées et des préadipocytes sont des cellules indifférenciées car non totalement différenciées car engagées dans un processus qui n'est pas arrivé à son terme.

Il en va de même des fibroblastes, qui sont bien des cellules indifférenciées comme cela est indiqué dans le document «Altération de la différenciation adipogénique des fibroblastes dermiques avec l'âge », Annales de Dermatologie et de Vénéréologie Volume 143, Issue 12, Supplement, December 2016, Page S432) qui conclut que « *les fibroblastes, de par leur capacité à se différencier en adipocytes, contribuent au renouvellement du tissu adipeux* ». Enfin pour ce qui est des cellules de la fraction du stroma vasculaire, elles constituent aussi des cellules indifférenciées, au sens de la présente invention, comme indiqué dans « Le tissu adipeux : Monsieur Hyde Obésité et Docteur Jekyll Médecine régénérative » (Med Sci (Paris). 2006 November; 22(11): 928-929) lorsque les auteurs indiquent que « *...la fraction stromale du tissu adipeux contient un gradient de cellules progénitrices ayant différentes potentialités de différenciation...* ».

Ces cellules indifférenciées sont généralement des cellules multipotentes, en particulier ce sont des cellules souches mésenchymateuses. Les cellules mésenchymateuses peuvent donner des adipocytes, des ostéocytes et des chondrocytes.

Elles sont capables de donner naissance à différentes lignées cellulaires d'un tissu donné. Ces cellules souches multipotentes sont présentes dans l'embryon ou dans l'organisme adulte, elles sont à l'origine de plusieurs types de cellules différenciées mais conservent leur capacité à s'autorenouveler.

Les cellules souches mésenchymateuses peuvent donner naissance à plusieurs types de cellules, et elles sont déjà engagées dans une certaine direction. Leurs potentialités sont donc plus restreintes que celles des cellules souches embryonnaires. Les cellules hématopoïétiques des mammifères, par exemple, donnent des globules rouges, des plaquettes, des lymphocytes T ou B, des macrophages, mais elles ne peuvent pas donner des cellules musculaires. Un autre exemple de cellules souches multipotentes est apporté par les cellules de la crête neurale qui émigrent du tube neural au cours de l'embryogénèse et qui donnent notamment naissance aux mélanocytes, aux neurones et aux cellules gliales du système nerveux périphérique.

Selon un mode particulier de l'invention encore, les cellules indifférenciées sont des préadipocytes.

Les préadipocytes sont des cellules indifférenciées, peu différenciées découlant de cellules souches mésenchymateuses, et qui peuvent être stimulées pour donner des adipocytes qui sont des cellules totalement différenciées.

Selon un mode particulier de l'invention encore, les cellules indifférenciées sont des fibroblastes.

Selon un autre mode particulier de l'invention encore, les cellules indifférenciées sont des cellules de la fraction stroma vasculaire du tissu adipeux.

Selon un mode de réalisation de l'invention, les cellules indifférenciées proviennent d'un ou plusieurs individus humains, en particulier adultes.

Selon un mode de réalisation les cellules indifférenciées sont issues de biopsies ou plasties de tissu humain, plus particulièrement de tissu adipeux humain ou encore de produit de liposuccion. Selon un autre mode de réalisation il s'agit de tissu cutané, comprenant le derme. Ces produits sont typiquement classifiés comme déchets opératoires.

La méthode selon l'invention n'est donc pas une méthode médicale ou chirurgicale. Selon un mode particulier et préférentiel, les cellules indifférenciées ne sont pas des cellules indifférenciées embryonnaires.

L'étape a) du procédé selon l'invention prévoit l'ensemencement, dans un milieu de culture adapté, des cellules indifférenciées isolées et amplifiées, dans un contenant dont le fond à un profil arrondi concave.

Le milieu de culture adapté correspond à un milieu de culture permettant la survie des cellules et contient les éléments nutritifs spécifiques au type cellulaire.

Par exemple, un milieu de culture adapté peut être un milieu EMEM ou DEMEM supplémenté en sérum foetal, en particulier sérum de veau foetal (en particulier environ 10% en masse) ou ECBM supplémenté avec de la sérum albumine, en particulier bovine (par exemple environ 3% en masse). Il peut aussi s'agir de milieu riche pour culture de cellules de mammifère et de préférence tel qu'il contient les acides aminés tels que alanine, asparagine, acide glutamique, et proline ; les vitamines telles que B8 et B12, les acides gras tels que linoléique et lipoïque, et des bases azotées telle thymidine ; il peut s'agir de préférence du milieu DMEM/F12 commercialisé par la Société Life Technologies ou du milieu ECBM commercialisé par Promocell.

Dans le cas où les cellules indifférenciées sont des préadipocytes, le milieu de culture adapté est un milieu tel que EMEM ou DMEM enrichi en sérum foetal, en particulier du sérum de veau foetal. Un milieu EMEM ou Eagle's minimal essential medium (EMEM) ou milieu minimum essentiel de Eagle, est un milieu de culture cellulaire mis au point par Harry Eagle et utilisé pour l'apport d'éléments nutritifs nécessaires au maintien et à la prolifération de différents types de cellules in vitro. Un tel milieu contient des acides aminés, des sels (chlorure de potassium, sulfate de magnésium, chlorure de sodium et du dihydrogénophosphate de sodium), du glucose et des vitamines (acide folique, nicotinamide, riboflavine et vitamine B12 par exemple). Une variation du EMEM existe, il s'agit du DMEM (pour Dulbecco/Vogt modified Eagle's minimal essential medium). Il contient approximativement quatre fois plus de vitamines et d'acides aminés que dans la formule originale et de deux à quatre fois plus de glucose. En outre, il peut contenir du fer également. Le milieu DMEM est approprié pour presque tous les types de cellules, comprenant les cellules humaines, de singe, de hamster, de rat, de souris, de volaille et de poisson.

Le contenant de l'étape a) est un contenant dont le fond présente un profil arrondi concave. Les inventeurs ont en effet constaté que la formation des sphéroïdes via les étapes b) et c) de centrifugation, ainsi que la répétition de ces étapes, était obtenue par la combinaison de cette alternance centrifugation/rotation du contenant avec le profil arrondi concave du contenant d'ensemencement.

A titre d'exemple un tel contenant dont le fond présente un profil arrondi concave correspond à un puits tel que ceux que l'on trouve sur les plaques de culture cellulaire comprenant une pluralité de puits, en particulier les plaques de culture 96 puits. On peut citer de manière avantageuse les plaques de culture microplaques Corning^{®} ULA comprenant 96 ou 384 puits par exemple.

De manière avantageuse, le contenant est traité « low attachment » ou « ultra-low attachment » afin de réduire l'adhésion cellulaire.

L'étape b) du procédé selon l'invention comprend une centrifugation dudit contenant. L'étape c) comprend une rotation du contenant, d'un angle de 160 à 200°, avantageusement 180°, selon un axe passant par le plan défini par la surface du milieu de culture contenu dans le contenant et perpendiculaire à celui-ci, .

Ces deux étapes b) et c) sont répétées entre 1 et 15 fois, par exemple 1 fois, 2 fois, 3 fois, 4 fois, 5 fois, 6 fois, 7 fois, 8 fois, 9 fois, 10 fois, 11 fois, 12 fois, 13 fois, voire 14 fois ou 15 fois.

De manière avantageuse, le nombre de répétition des étapes b) et c) est de trois, voire de 4, voire encore de 5, voire 10 fois, particulièrement 13 fois.

Comme expliqué, entre chaque centrifugation, le contenant, ou encore préférentiellement la plaque de culture cellulaire comprenant une pluralité de puits, subit une rotation d'un angle de l'ordre de 160 à 200°, plus particulièrement de 180°, selon un axe perpendiculaire au plan défini par la surface du milieu de culture qui est contenu dans ledit contenant, l'axe de rotation passant par ledit plan.

Cette centrifugation peut être réalisée à l'aide d'une centrifugeuse de paillasse, particulièrement une centrifugeuse pour plaque de culture cellulaire comprenant une pluralité de puits.

A titre d'exemple on peut citer les centrifugeuses de microplaques, par exemple une centrifugeuse munie d'un rotor vertical à double position, qui prend en charge une vaste gamme de plaques. Le rotor dispose de guides de plaques pour maintenir les plaques. On peut aussi citer la centrifugeuse de la société MBI, PlateFuge^{®} MicroCentrifuge pour plaques. C'est une centrifugeuse avec un rotor swing pour des microplaques. La chambre du rotor facilement accessible comprend deux supports de plaques. Cela permet aux plaques d'être déposées sur chaque support. Lors de la fermeture du couvercle de la centrifugeuse, le rotor accélère et la force centrifuge permet de plaquer les plaques dans une position verticale.

Le procédé consiste à prendre le contenant, par exemple une plaque 96 puits à fond arrondi, à la disposer dans le support Adhoc de la centrifugeuse, de faire une première centrifugation pendant quelques minutes. Apres cette première centrifugation, la plaque est ôtée de son support, on procède à une rotation de la plaque - par exemple de 180° - sur elle-même avant de la disposer à nouveau dans le support Adhoc puis de faire une seconde centrifugation. Ces étapes sont ainsi répétées 3, 4 voire par exemple encore 5 fois, avec, entre chaque centrifugation une rotation de la plaque sur elle-même ; c'est-à-dire en l'ôtant du support, en lui faisant faire une rotation, puis en la remettant dans le support avant de relancer la centrifugation suivante.

L'étape de centrifugation dure en moyenne quelques minutes, en particulier entre 1 et 10 minutes, en particulier entre 1 et 5 minutes, plus particulièrement encore entre 1 et 3 minutes.

La force de centrifugation est comprise entre 100 et 500 g pendant la durée de la centrifugation, en particulier aux environ de 200 à 400 g, plus particulièrement 200 à 300 g environ.

Selon un mode particulier de réalisation de l'invention, les étapes b) et c) de centrifugation/rotation sont répétées 2 à 10 fois, voire encore 2 à 8 fois, plus particulièrement encore 2 à 6 fois, voire plus particulièrement 2 à 4 fois.

La combinaison de répétitions de centrifugations avec une rotation du contenant, i.e. de la plaque, entre chaque centrifugation, associées au fond arrondi convexe permet la formation des sphéroïdes et ce sans ajout d'agent chimique, de support matriciel protéique ou polysaccharidique. De surcroit, la formation des sphéroïdes ne nécessite pas plusieurs jours ; elle est réalisée en quelques minutes, voire quelques heures.

Les sphéroïdes néo-formés sont ensuite ensemencés. L'étape e) vise ainsi l'ensemencement et l'incubation des sphéroïdes de cellules indifférenciées néo formés dans un milieu supplémenté en sérum foetal, en particulier du sérum de veau foetal,favorisant la formation complète de sphéroïdes. Un tel milieu peut être un milieu EMEM ou DMEM supplémenté en sérum foetal, en particulier sérum de veau foetal (en particulier environ 10% en masse).

De manière avantageuse, que les conditions de l'étape e) d'incubation des sphéroïdes de de cellules indifférenciées néo formés correspondent à une température comprise entre 36°C et 38°C et préférentiellement aux alentours de 37°C et à un pourcentage de CO2 compris entre 3 et 6 % et préférentiellement aux alentours de 5%.

La formation complète des sphéroïdes est contrôlée par l'évaluation de la forme, de l'intégrité et de la croissance des sphéroïdes par imagerie en plaque en lumière transmise et mesure de l'aire (selon méthode décrite dans « Scaffold-free génération of uniform adipose spheroids for metabolism research and drug discovery ». Klingelhutz A.J. et al. 2018 ; Nature Scientific Reports - 8 :523.).

Selon un mode de réalisation, le procédé selon l'invention vise l'obtention de sphéroïdes de cellules différenciées comprenant les étapes a à e citées plus avant et comprend en outre les étapes supplémentaires suivantes :
- f. Différenciation des sphéroïdes de cellules indifférenciées totalement formés en sphéroïdes de cellules différenciées dans un milieu de différenciation,
- g. Obtention des sphéroïdes de cellules différenciées.

L'étape de différenciation est effectuée selon les techniques classiques et connues de l'homme du métier et ce selon le type cellulaire.

Selon un mode particulier de réalisation, le procédé selon l'invention est caractérisé en ce que les cellules indifférenciées sont des pré-adipocytes, que les cellules différenciées sont des adipocytes et en ce que le milieu de l'étape e) conduisant à la différenciation des sphéroïdes de pré-adipocytes en sphéroïdes d'adipocytes comprend un tampon, un corticoïde, un inhibiteur de phosphodiestérase, un antidiabétique, un capteur de fer, et au moins une hormone.

De manière particulière, le milieu est un mélange d'Hépès, d'insuline, de T3, de Dexaméthasone, de transferrine, de rosiglitazone et d'IBMX.

Ainsi, dans un mode de réalisation particulier où le procédé selon l'invention vise à l'obtention de sphéroïdes d'adipocytes, le procédé selon l'invention est caractérisé en ce que le tampon est choisi dans le groupe comprenant le bicarbonate et l'hépès, préférentiellement l'hépès et à une concentration comprise entre 5 et 30 mM, préférentiellement entre 10 et 20 mM.

Selon un mode de réalisation particulier où le procédé selon l'invention vise à l'obtention de sphéroïdes d'adipocytes, le procédé selon l'invention est caractérisé en ce que l'hormone est choisie dans le groupe comprenant l'insuline et l'hormone thyroïdienne T3, seule ou en mélange, préférentiellement en mélange, à des concentrations comprises entre 10 et 100nM, préférentiellement entre 30 et 60 nM pour l'insuline et entre 0.1 et 2nM, préférentiellement entre 0.5 et 1.5nM pour l'hormone thyroïdienne T3.

Selon un mode de réalisation particulier où le procédé selon l'invention vise à l'obtention de sphéroïdes d'adipocytes, le procédé selon l'invention est caractérisé en ce que le corticoïde est choisi dans le groupe comprenant bétaméthasone, dexamethasone cortivazol, et leurs mélanges, préférentiellement la Dexamethasone à une concentration finale comprise entre 50 et 200 nM, préférentiellement entre 80 et 120nM.

Selon un mode de réalisation particulier où le procédé selon l'invention vise à l'obtention de sphéroïdes d'adipocytes, le procédé selon l'invention est caractérisé en ce que le capteur/transporteur de fer, notamment la transferrine, est à une concentration comprise entre 0.01 et 0.2 µg/ml, préférentiellement entre 0.05 et 0.15µg/ml.

Selon un mode de réalisation particulier où le procédé selon l'invention vise à l'obtention de sphéroïdes d'adipocytes, le procédé selon l'invention est caractérisé en ce que l'antidiabétique est choisi dans le groupe comprenant pioglitazone, rosiglitazone, troglitazone, ainsi que leurs mélanges, préférentiellement le rosiglitazone à une concentration finale comprise entre 50 et 200nM, préférentiellement entre 80 et 120 nM. Selon un mode de réalisation particulier où le procédé selon l'invention vise à l'obtention de sphéroïdes d'adipocytes, le procédé selon l'invention est caractérisé en ce que l'inhibiteur de phosphodiesterase non sélectif est choisi dans le groupe comprenant la caféine, la théophylline, le 3-isobutyl-1-methylxantine (IBMX) et leurs mélanges, préférentiellement l'IBMX à une concentration comprise entre 5 et 35µM, préférentiellement entre 15 et 25µm. Les sphéroïdes obtenus présentent un diamètre compris entre 80 et 200 µm, voire entre 100 et 200 µm, particulièrement entre 110 et 190 µm, plus particulièrement entre 120 et 160 µm et ce selon le stade de maturité et/ou de différenciation.

En effet les sphéroïdes obtenus selon l'invention constituent un modèle d'étude optimal car réalisé à partir de cultures primaires de cellules, c'est-à-dire des cellules qui sont issues directement de tissus humains frais, et placées dans des conditions qui miment au mieux les conditions physiologiques que l'on retrouve *in vivo*, dans le corps humain. La taille des sphéroïdes permet la diffusion de l'oxygène, et autres gaz ainsi que des nutriments jusqu'au coeur du sphéroïde, ce qui permet de maintenir toutes les cellules depuis la périphérie jusqu'au centre en état d'oxygénation et de nutrition adéquat le plus proche possible des conditions in vivo. Avec des sphéroïdes ou particules (non sphériques) de plusieurs centaines de µm, des cellules se nécrosent par défaut d'oxygénation ou les nutriments ne les atteignent pas. Ceci s'oppose aux modèles mettant en oeuvre des cellules dites « immortalisées ». Le métabolisme de ces cellules est modifié artificiellement pour faciliter leur manipulation et la variabilité des résultats, ce qui, en revanche, engendre des caractéristiques différentes, plus éloignées du vivant et introduit un biais dans les études. En outre, l'absence de matrice exogène pour la formation de ces sphéroïdes renforce encore cet avantage technique et biologique puisque toute étude sur les processus de remodelage matriciel de ces cellules ne sera pas biaisée par des composants étrangers ajoutés pour la formation des sphéroïdes.

Les sphéroïdes obtenus selon l'invention peuvent être avantageusement utilisés pour des protocoles de criblage biologique et de produits c'est-à-dire soit pour mettre en évidence un effet biologique d'un produit donné soit pour identifier un produit d'intérêt à partir d'une banque de produits test. Comme évoqué plus avant leur forme et leur taille permet au produit à tester d'atteindre toutes les cellules et les résultats obtenus sont d'autant plus proche de la réalité physiologique in vivo.

L'invention vise ainsi l'utilisation des sphéroïdes ainsi obtenus selon l'invention aux fins d'évaluation de composés test.

La présente invention fournit ainsi un procédé d'évaluation de propriétés pharmacologiques de composés tests comprenant les étapes suivantes :
- a. mettre en oeuvre un procédé selon l'invention afin d'obtenir des sphéroïdes de cellules différenciées;
- b. Mettre en contact un composé test avec les sphéroïdes ;
- c. Evaluer l'impact du composé test sur au moins un paramètre physiologique ou morphologique des cellules des sphéroïdes.

De manière particulièrement avantageuse, les sphéroïdes selon l'invention permettent des études de composés tests dans différents domaines, qu'il s'agisse du secteur cosmétique pour évaluer des composés ou actifs visant à la gestion du poids et de la silhouette, des produits anti-âge, anti-pollution ou encore axés sur la qualité de la peau et des cheveux. Est aussi concerné le secteur de la nutrition santé pour ce qui concerne les compléments alimentaires aux propriétés similaires à celles concernant le domaine cosmétique ainsi que les produits concernant la nutrition sportive ou encore les défenses immunitaires.

Enfin, le secteur pharmaceutique est aussi particulièrement visé avec les produits ciblant les phénomènes de surcharges pondérales, d'obésité ou encore les troubles métaboliques associés comme le diabète par exemple.

De manière avantageuse encore, les sphéroïdes selon l'invention peuvent être utilisés dans le domaine de la médecine esthétique afin d'évaluer de produits de comblement tels que des fillers comprenant des polymères organiques polysaccharidiques ou protéiques par exemple.

### Description des Figures :

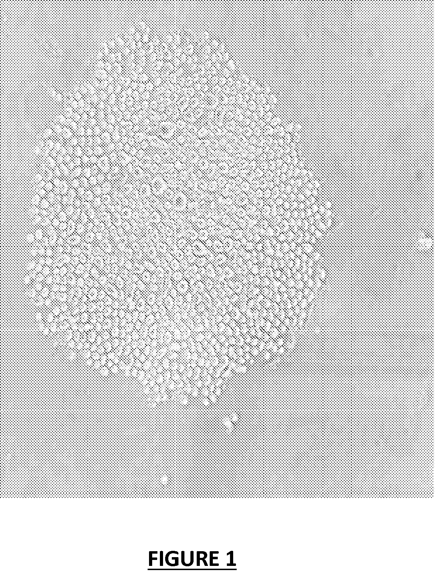

La FIGURE 1 montre une photo de sphéroïdes obtenus selon le procédé de l'exemple 1, après l'étape 7, c'est-à-dire le surlendemain de l'ensemencement, juste avant de débuter la différenciation.

### Exemples :

EXEMPLE de Procédé d'obtention de sphéroïdes de préadipocytes :
PRINCIPE :
Ensemencer les préadipocytes humains isolés du tissu adipeux pour former des sphéroïdes.

MATERIELS :
- Plaque 96 puits fond rond (7007 - Costar)
- Milieu de culture des préadipocytes 37°C *(DMEM 10% Sérum de Veau Foetal (SVF) 1%* PS)
- Microscope

Centrifugeuse
ABREVIATIONS :
DMEM : Dulbecco's Modified Eagle Medium
AU PREALABLE :
Les préadipocytes sont isolés à partir de tissu adipeux humain issu de biopsies ou plasties de tissu adipeux humain ou encore de produit de liposuccion. Le tissu est découpé et broyé ou aspiré avec une pipette à bout cassé afin d'assurer séparation des cellules. Une solution de collagénase est ajoutée pour faciliter la séparation des cellules. Les cellules sont prélevées en évitant le sang et/ou la couche d'huile qui pourrait s'être formée suite à éclatement des adipocytes. Les cellules sont lavées avec du PBS et subissent plusieurs centrifugations/lavages, aux environs de 300 à 500 g, pour éliminer débris cellulaires et globules rouges. Les cellules sont lavées avec du PBS. Après deux centrifugations/lavages, le culot de cellules est mis en suspension dans le milieu de culture de préadipocytes. Les cellules sont ensuite ensemencées en flasque T175 à raison de 5 à 10 millions de cellules par flasque. Le lendemain, les cellules sont ensuite lavées avec du PBS et remises en culture avec le milieu de culture de préadipocytes. A confluence, les cellules sont traitées à la trypsine pour bien les décoller ; puis du milieu de culture de préadipocytes est ajouté pour neutraliser l'action de l'enzyme et les cellules remises en suspension. Elles sont prêtes pour le protocole d'obtention de sphéroïdes.

### PROTOCOLE POUR OBTENTION SPHEROIDES :

1- Dans une plaque 96 puits fond rond, ensemencer les préadipocytes à 1000 cellules par puits dans 150 µL de milieu DMEM 10% SVF
2- Centrifuger la plaque à 200g pendant 2 minutes à température ambiante
3- Tourner la plaque sur elle-même de 180° et
4- répéter l'étape 2) et l'étape 3) 4 fois
5- Après les centrifugations, vérifier la bonne agrégation des préadipocytes (début de formation du sphéroïde) et incuber à 37°C 5% CO2 pendant une nuit
6- Le lendemain, vérifier la bonne formation des sphéroïdes
7- Le surlendemain de l'ensemencement, débuter le traitement de différenciation (Tableau 1 ci-dessous pour la préparation du milieu de base et le Tableau 2 ci-dessous pour le milieu de différenciation). Le milieu est déposé doucement au bord du puits pour ne pas désintégrer le sphéroïde.

**Tableau 1 : Milieu de base pour la différenciation.**

| [Table 1] | |
|---|---|
| | Concentration finale |
| DMEM/F12 sans rouge de phénol | QSP |
| Biotine | 33 µM |
| Calcium Panthoténate | 17 µM |
| Pénicilline - Streptomycine | 1% (100U/mL) |
| Glutamine stabilisée | 2,5mM |

**Tableau 2 : Milieu de différenciation**

| [Table 2] | | |
|---|---|---|
| | Volume | Concentration finale dans le puits |
| Milieu de base | 10 mL | |
| Hépès | | 10 à 30 mM |
| Insuline | | 30 à 80 nM |
| T3 | | 0.5 à 2 nM |
| Dexaméthasone | | 80 à 150 nM |
| Transferrine | | 0.05 à 2 µg/ml |
| Rosiglitazone | | 50 à 120 nM |
| IBMX | | 10 à 30 µM |

7- Changer les milieux tous les 2-3 jours et observer les sphéroïdes tous les jours pour vérifier leur bonne tenue.

8- Arrêter la différenciation si les sphéroïdes commencent à se désagréger.

Les sphéroïdes de cellules préadipocytes non différenciées obtenus et comprenant environ 1000 cellules sont visibles sur la FIGURE 1. On note une formation bien sphérique de l'amas de cellules.

Le Tableau 3 ci-dessous illustre la taille (diamètre en µm) des sphéroïdes obtenus selon le procédé de l'exemple, avec des quantités variables de cellules à l'ensemencement, à la fin du procédé (3 jours après ensemencement) et quelques jours après (7 jours après ensemencement).

**Tableau 3 : Taille de sphéroïdes obtenus selon le procédé (diamètre en µm et écart type) et selon le nombre de cellules à l'ensemencement et après 3 ou 7 jours post-ensemencement.**

| | | Diamètre en µm (Nombre de cellules à l'ensemencement) | | | | | | | |
|---|---|---|---|---|---|---|---|---|---|
| | | Diamètre (1-1000) | Ecart-type | Diamètre (2-1000) | Ecart-type | Diamètre (1-2000) | Ecart-type | Diamètre (2-2000) | Ecart-type |
| Jours post-ensemencement | 3 | 126 | 7.5 | 120 | 7 | 150 | 8 | 155 | 8.5 |
| | 7 | 110 | 5.2 | 124 | 5 | 128 | 10 | 140 | 9.5 |

## Revendications

1. Procédé d'obtention de sphéroïdes de cellules indifférenciées comprenant les étapes suivantes :
a. Ensemencement de cellules indifférenciées isolées et amplifiées dans un contenant dont le fond a un profil arrondi concave, contenant un milieu de culture adapté,
b. Centrifugation dudit contenant,
c. Rotation du contenant selon un axe passant perpendiculairement au plan défini par la surface du milieu de culture, la rotation étant de de 160 à 200°, avantageusement 180°,
d. répétition des étapes b) et c) entre 1 et 15 fois,
e. Ensemencement et incubation des sphéroïdes de cellules indifférenciées néo formées dans un milieu favorisant la formation complète de sphéroïdes, en particulier un milieu supplémenté en sérum foetal,
**caractérisé en ce que** lesdites cellules indifférenciées ne sont pas des cellules embryonnaires humaines.

2. Procédé d'obtention de sphéroïdes de cellules différenciées comprenant les étapes a) à e) du procédé selon la revendication 1 et comprenant en outre les étapes supplémentaires suivantes :
- f. Différenciation des sphéroïdes de cellules indifférenciées totalement formés en sphéroïdes de cellules différenciées dans un milieu de différenciation,
- g. Obtention des sphéroïdes de cellules différenciées.

3. Procédé selon la revendication 1 ou 2 **caractérisé en ce que** les cellules indifférenciées ont été prélevées chez un ou plusieurs individus humains.

4. Procédé selon la revendication 1 ou 2 **caractérisé en ce que** le contenant est un puits à fond de profil arrondi concave compris dans un dispositif de culture tel qu'une plaque comprenant une pluralité de puits.

5. Procédé selon l'une des revendications précédentes **caractérisé en ce que** la concentration des cellules ensemencées dans le contenant correspond à une quantité de cellules par contenant comprise entre 500 et 1500, avantageusement 1000.

6. Procédé selon l'une des revendications précédentes **caractérisé en ce que** la durée de chaque centrifugation est comprise entre 1 à 3 minutes, préférentiellement aux alentours de 2 minutes avec une force de centrifugation comprise entre 200 et 500g, préférentiellement aux alentours de 200g.

7. Procédé selon l'une des revendications précédentes **caractérisé en ce que** les conditions de l'étape e) d'incubation des sphéroïdes de cellules indifférenciées néo formés correspondent à une température comprise entre 36°C et 38°C et préférentiellement aux alentours de 37°C et à un pourcentage de CO2 compris entre 3 et 6 % et préférentiellement aux alentours de 5%.

8. Procédé selon l'une des revendications 1 à 7, **caractérisé en ce que** les cellules indifférenciées sont choisies dans le groupe consistant en pré-adipocytes, fibroblastes et cellules précurseurs des cellules de la fraction stroma vasculaire du tissu adipeux.

9. Procédé selon l'une des revendications 2 à 8, **caractérisé en ce que** les cellules indifférenciées sont des pré-adipocytes, que les cellules différenciées sont des adipocytes et **en ce que** le milieu de l'étape f) conduisant à la différenciation des sphéroïdes de pré-adipocytes en sphéroïdes d'adipocytes comprend un tampon, un corticoïde, un inhibiteur de phosphodiesterase, un antidiabétique, un capteur de fer, et au moins une hormone.

10. Procédé selon la revendication 9 **caractérisé en ce que** le tampon est choisi dans le groupe comprenant le bicarbonate et l'hépès, préférentiellement l'hépès et à une concentration comprise entre 5 et 30 mM, préférentiellement entre 10 et 20 mM.

11. Procédé selon la revendication 9 **caractérisé en ce que** l'hormone est choisie dans le groupe comprenant l'insuline et l'hormone thyroïdienne T3, seule ou en mélange, préférentiellement en mélange, à des concentrations comprises entre 10 et 100nM, préférentiellement entre 30 et 60 nM pour l'insuline et entre 0.1 et 2nM, préférentiellement entre 0.5 et 1.5nM pour l'hormone thyroïdienne T3.

12. Procédé selon la revendication 9 **caractérisé en ce que** le corticoïde est choisi dans le groupe comprenant bétaméthasone, dexamethasone cortivazol, et leurs mélanges, préférentiellement la Dexamethasone à une concentration finale comprise entre 50 et 200 nM, préférentiellement entre 80 et 120nM.

13. Procédé selon la revendication 9 **caractérisé en ce que** le capteur/transporteur de fer, notamment la transferrine, est à une concentration comprise entre 0.01 et 0.2 µg/ml, préférentiellement entre 0.05 et 0.15µg/ml.

14. Procédé selon la revendication 9 **caractérisé en ce que** l'antidiabétique est choisi dans le groupe comprenant pioglitazone, rosiglitazone, troglitazone, ainsi que leurs mélanges préférentiellement le rosiglitazone à une concentration finale comprise entre 50 et 200nM, préférentiellement entre 80 et 120 nM.

15. Procédé selon la revendication 9 **caractérisé en ce que** l'inhibiteur de phosphodiesterase non sélectif est choisi dans le groupe comprenant la caféine, la théophylline, le 3-isobutyl-1-methylxantine (IBMX) et leurs mélanges, préférentiellement l'IBMX à une concentration comprise entre 5 et 35 µM préférentiellement entre 15 et 25µM.

16. Procédé d'évaluation de propriétés pharmacologiques de composés test comprenant les étapes suivantes :
a. mettre en oeuvre un procédé selon l'une des revendications 2 à 9 afin d'obtenir des sphéroïdes de cellules différenciées;
b. mettre en contact un composé test avec les sphéroïdes ;
c. évaluer l'impact du composé test sur au moins un paramètre physiologique ou morphologique des cellules des sphéroïdes.

## Patentansprüche

1. Verfahren zur Gewinnung von Sphäroiden aus undifferenzierten Zellen, das die folgenden Schritte umfasst:
a. Aussäen von isolierten und amplifizierten undifferenzierten Zellen in einen Behälter, dessen Boden ein konkav abgerundetes Profil aufweist und der ein geeignetes Kulturmedium enthält,
b. Zentrifugieren dieses Behälters,
c. Drehen des Behälters um eine Achse, die senkrecht zu der durch die Oberfläche des Kulturmediums definierten Ebene verläuft, wobei die Drehung 160 bis 200°, vorteilhafterweise 180°, beträgt,
d. Wiederholen der Schritte b) und c) zwischen 1- und 15-mal,
e. Aussäen und Inkubieren der neu gebildeten Sphäroide aus undifferenzierten Zellen in einem Medium, das die vollständige Bildung von Sphäroiden fördert, insbesondere in einem Medium, das mit fetalem Serum angereichert ist,
**dadurch gekennzeichnet, dass** es sich bei den undifferenzierten Zellen nicht um menschliche embryonale Zellen handelt.

2. Verfahren zur Gewinnung von Sphäroiden aus differenzierten Zellen, das die Schritte a) bis e) des Verfahrens nach Anspruch 1 und darüber hinaus die folgenden zusätzlichen Schritte umfasst:
f. Differenzieren der vollständig gebildeten Sphäroide aus undifferenzierten Zellen zu Sphäroiden aus differenzierten Zellen in einem Differenzierungsmedium,
g. Gewinnen der Sphäroide aus differenzierten Zellen.

3. Verfahren nach Anspruch 1 oder 2, **dadurch gekennzeichnet, dass** die undifferenzierten Zellen einem oder mehreren menschlichen Individuen entnommen wurden.

4. Verfahren nach Anspruch 1 oder 2, **dadurch gekennzeichnet, dass** der Behälter eine Vertiefung mit einem konkaven abgerundeten Bodenprofil ist, die in einer Kulturvorrichtung wie einer Platte enthalten ist, die eine Vielzahl von Vertiefungen umfasst.

5. Verfahren nach einem der vorstehenden Ansprüche, **dadurch gekennzeichnet, dass** die Konzentration der in den Behälter ausgesäten Zellen einer Zellmenge pro Behälter zwischen 500 und 1500, vorteilhafterweise 1000, entspricht.

6. Verfahren nach einem der vorstehenden Ansprüche, **dadurch gekennzeichnet, dass** die Dauer jeder Zentrifugation zwischen 1 und 3 Minuten, vorzugsweise bei etwa 2 Minuten, mit einer Zentrifugationskraft zwischen 200 und 500 g, vorzugsweise um 200 g, liegt.

7. Verfahren nach einem der vorstehenden Ansprüche, **dadurch gekennzeichnet, dass** die Bedingungen von Schritt e) des Inkubierens der neu gebildeten Sphäroide aus undifferenzierten Zellen einer Temperatur zwischen 36°C und 38°C und vorzugsweise um 37°C und einem CO2-Anteil zwischen 3 und 6% und vorzugsweise um 5% entsprechen.

8. Verfahren nach einem der Ansprüche 1 bis 7, **dadurch gekennzeichnet, dass** die undifferenzierten Zellen ausgewählt sind aus der Gruppe bestehend aus Präadipozyten, Fibroblasten und Vorläuferzellen der Zellen der vaskulären Stromafraktion des Fettgewebes.

9. Verfahren nach einem der Ansprüche 2 bis 8, **dadurch gekennzeichnet, dass** die undifferenzierten Zellen Präadipozyten sind, dass die differenzierten Zellen Adipozyten sind und dass das Medium von Schritt f), das zur Differenzierung der Sphäroide von Präadipozyten zu Sphäroiden von Adipozyten führt, einen Puffer, ein Kortikoid, einen Phosphodiesterase-Hemmer, ein Antidiabetikum, einen Eisenfänger und mindestens ein Hormon umfasst.

10. Verfahren nach Anspruch 9, **dadurch gekennzeichnet, dass** der Puffer ausgewählt ist aus der Gruppe bestehend aus Bicarbonat und HEPES, vorzugsweise HEPES, und in einer Konzentration zwischen 5 und 30 mM, vorzugsweise zwischen 10 und 20 mM.

11. Verfahren nach Anspruch 9, **dadurch gekennzeichnet, dass** das Hormon ausgewählt ist aus der Gruppe bestehend aus Insulin und dem Schilddrüsenhormon T3, allein oder in Mischung, vorzugsweise in Mischung, in Konzentrationen zwischen 10 und 100 nM, vorzugsweise zwischen 30 und 60 nM, für Insulin und zwischen 0,1 und 2 nM, vorzugsweise zwischen 0,5 und 1,5 nM, für das Schilddrüsenhormon T3.

12. Verfahren nach Anspruch 9, **dadurch gekennzeichnet, dass** das Kortikoid ausgewählt ist aus der Gruppe bestehend aus Betamethason, Dexamethason Cortivazol und Mischungen davon, vorzugsweise Dexamethason in einer Endkonzentration zwischen 50 und 200 nM, vorzugsweise zwischen 80 und 120 nM.

13. Verfahren nach Anspruch 9, **dadurch gekennzeichnet, dass** der Eisenfänger bzw. -transporter, insbesondere Transferrin, in einer Konzentration zwischen 0,01 und 0,2 µg/ml, vorzugsweise zwischen 0,05 und 0,15 µg/ml, vorliegt.

14. Verfahren nach Anspruch 9, **dadurch gekennzeichnet, dass** das Antidiabetikum ausgewählt ist aus der Gruppe bestehend aus Pioglitazon, Rosiglitazon, Troglitazon sowie Mischungen davon, vorzugsweise Rosiglitazon, in einer Endkonzentration zwischen 50 und 200 nM, vorzugsweise zwischen 80 und 120 nM.

15. Verfahren nach Anspruch 9, **dadurch gekennzeichnet, dass** der nicht-selektive Phosphodiesterase-Hemmer ausgewählt ist aus der Gruppe bestehend aus Koffein, Theophyllin, 3-Isobutyl-1-methylxanthin (IBMX) und Mischungen davon, vorzugsweise IBMX, in einer Konzentration zwischen 5 und 35 µM, vorzugsweise zwischen 15 und 25 µM.

16. Verfahren zur Bewertung der pharmakologischen Eigenschaften von Testverbindungen, das die folgenden Schritte umfasst:
a. Durchführen eines Verfahrens nach einem der Ansprüche 2 bis 9, um Sphäroide aus differenzierten Zellen zu gewinnen;
b. Inkontaktbringen einer Testverbindung mit den Sphäroiden;
c. Bewerten der Auswirkungen der Testverbindung auf mindestens einen physiologischen oder morphologischen Parameter der Zellen der Sphäroide.

## Claims

1. Method for obtaining spheroids of undifferentiated cells comprising the following steps:
a. Inoculating undifferentiated isolated and amplified undifferentiated cells into a container whose bottom has a rounded concave profile, containing a suitable culture medium,
b. Centrifuging said container,
c. Rotating the container along an axis perpendicular to the plane defined by the surface of the culture medium, the rotation being from 160 to 200°, advantageously 180°.
d. Repeating steps b) and c) between 1 and 15 times,
e. Inoculating and incubating the newly formed spheroids of undifferentiated cells into a medium promoting complete spheroid formation, especially a medium supplemented with foetal serum.
**characterized in that** said undifferentiated cells are not human embryonic cells.

2. Method for obtaining spheroids of differentiated cells comprising steps a) to e) of the method according to claim 1 and also comprising the following additional steps:
- f. Differentiating the fully formed undifferentiated cell spheroids into differentiated cell spheroids in a differentiation medium,
- g. Obtaining spheroids of differentiated cells.

3. Method according to claim 1 or 2, **characterized in that** the undifferentiated cells are drawn from one or more individual humans.

4. Method according to claim 1 or 2, **characterized in that** the container is a well with a concave rounded bottom profile comprised in a cell culture device such as a plate comprising a plurality of wells.

5. Method according to one of the preceding claims, **characterized in that** the concentration of cells inoculated into the container corresponds to a quantity of cells per container comprised between 500 and 1500, advantageously 1000.

6. Method according to one of the preceding claims, **characterized in that** the duration of each centrifugation is between 1 and 3 minutes, preferentially around 2 minutes with a centrifugation force comprised between 200 and 500 g, preferably around 200 g.

7. Method according to one of the preceding claims, **characterized in that** the conditions of step e) of incubation of the newly formed undifferentiated cell spheroids correspond to a temperature comprised between 36°C and 38°C and preferentially around 37°C and to a percentage of CO₂ between 3 and 6% and preferentially around 5%.

8. Method according to one of claims 1 to 7, **characterized in that** the undifferentiated cells are chosen in the group consisting of preadipocytes, fibroblasts and precursor cells of the vascular stromal fraction of adipose tissue.

9. Method according to the one of claims 2 to 8, **characterized in that** the undifferentiated cells are preadipocytes, the differentiated cells are adipocytes and the medium of step f) leading to the differentiation of preadipocyte spheroids to adipocyte spheroids comprises a buffer, a corticosteroid, a phosphodiesterase inhibitor, an antidiabetic, an iron scavenger, and at least one hormone.

10. Method according to claim 9, **characterized in that** the buffer is chosen in the group comprising bicarbonate and Hepes, preferentially Hepes at a concentration comprised between 5 and 30 nM, preferentially between 10 and 20 mM.

11. Method according to the claim 9, **characterized in that** the hormone is chosen in the group comprising insulin and thyroid hormone T3, alone or in mixture, preferentially in mixture, at concentrations comprised between 10 and 100 nM, preferentially between 30 and 60 nM for insulin and between 0.1 and 2 nM, preferentially between 0.5 and 1.5 nM for thyroid hormone T3.

12. Method according to claim 9, **characterized in that** the corticosteroid is chosen in the group comprising betamethasone, dexamethasone cortivazol and mixtures thereof, preferentially Dexamethasone at a final concentration comprised between 50 and 200 nM, preferentially between 80 and 120 nM.

13. Method according to claim 9, **characterized in that** the iron scavenger/transporter, especially transferrin, is at a concentration comprised between 0.01 and 0.2 µg/ml, preferentially between 0.05 and 0.15 µg/ml.

14. Method according to claim 9, **characterized in that** the antidiabetic is chosen in the group comprising pioglitazone, rosiglitazone, troglitazone and mixtures thereof, preferentially rosiglitazone at a final concentration comprised between 50 and 200 nM, preferentially between 80 and 120 nM.

15. Method according to claim 9, **characterized in that** the non-selective phosphodiesterase inhibitor is selected from the group comprising caffeine, theophylline, 3-isobutyl-1-methylxantine (IBMX) and mixtures thereof, preferentially IBMX at a concentration comprised between 5 and 35 µM), preferentially between 15 and 25µm.

16. Method for evaluating the pharmacological properties of test compounds comprising the following steps:
a. Implementing a method according to one of claims 2 to 9 in order to obtain spheroids of differentiated cells;
b. Contacting a test compound with the spheroids;
c. Evaluating the impact of the test compound on at least one physiological or morphological parameter of the cells of the spheroids.
